Europäisches Patentamt

European Patent Office

Office européen des brevets

(19)

(11) Veröffentlichungsnummer: **0 071 269**
**B1**

(12) **EUROPÄISCHE PATENTSCHRIFT**

(45) Veröffentlichungstag der Patentschrift:
01.03.89

(51) Int. Cl.⁴: **A 61 B 5/10, H 01 L 27/20**

(21) Anmeldenummer: 82106868.1

(22) Anmeldetag: 29.07.82

(54) Fingerabdrucksensor zur Transformation eines topologischen Musters eines zu untersuchenden Fingers in ein elektrisches Ausgangssignal.

(30) Priorität: 30.07.81 US 288288

(43) Veröffentlichungstag der Anmeldung:
09.02.83 Patentblatt 83/6

(45) Bekanntmachung des Hinweises auf die Patenterteilung:
01.03.89 Patentblatt 89/9

(84) Benannte Vertragsstaaten:
BE CH DE FR GB IT LI NL SE

(56) Entgegenhaltungen:
EP-A- 0 044 489
US-A- 3 569 747
US-A- 3 781 855
US-A- 3 808 473
US-A- 3 973 146
US-A- 4 250 894

IBM TECHNICAL DISCLOSURE BULLETIN, Band 14, Nr. 11, April 1972, Seite 3361, New York, US; R.P. JAMES: "Finger-print sensor"

(73) Patentinhaber: Siemens Aktiengesellschaft Berlin und München, Wittelsbacherplatz 2, D-8000 München 2 (DE)

(72) Erfinder: Edwards, David G., Dr., 838-B Country Club Parkway, 08054 Mount Laurel New Jersey (US)

## Beschreibung

Die vorliegende Erfindung betrifft gemäß Anspruch 1 einen Fingerabdrucksensor zur Transformation des topologischen Musters eines zu untersuchenden Fingers in ein elektrisches Ausgangssignal.

Systeme zur Identifikation von Fingerabdrükken, welche die Rippen- und Rillenkonfiguration eines auf eine Kontaktfläche gedrückten Fingers identifizieren, sind wohlbekannt.

In verschiedenen Systemen wird der Fingerabdruck von einem durch die Frontfläche eines transparenten Fingerbettes geleiteten Lichtstrahl abgefragt. Der abfragende Strahl wird an der Rückfläche entsprechend den optischen Diskontinuitäten, die durch den auf diese Fläche gedrückten Finger erzeugt werden, reflektiert. Folglich enthält das reflektierte Licht die Information des Fingerabdruckes, die von einem optoelektrischen Element empfangen werden kann. Ein derartiger Sensor geht beispielsweise aus der US-PS 4 053 228 hervor und erfordert Vorrichtungen zur Strahlabtastung und Fokussierung und ist deshalb relativ voluminös und kompliziert. Einrichtungen zur Strahlausrichtung können vermieden werden, wenn die Konstruktion in folgender Weise geändert wird: Strahlung wird durch ein plattenförmiges Fingerbett mittels innerer Totalreflexion geleitet. Die Platte erscheint einem Feld aus fotoempfindlichen Elementen, das sich in einer Ebene parallel zur Fingerbettplatte erstreckt, homogen dunkel. Wenn der Finger einmal auf die Platte gedrückt ist, wird die elastische oder nachgiebige Plattenfläche verformt und bewirkt ein Lichtmuster, welches dem aufgedrückten Relief entspricht. Diese Konstruktion ist in der US-Patentanmeldung Serial No. 176 696, angemeldet am 11. August 1980 vorgeschlagen und kann als eine aus nur wenigen Teilen zusammengesetzte Einheit mit geringen Abmessungen ausgeführt werden. Sie weist jedoch noch die allen mit Licht arbeitenden Sensoren gemeinsamen Unzulänglichkeiten auf: Die Notwendigkeit einer leistungsverbrauchenden Strahlungsquelle und optische Elemente, beispielsweise Filter oder Linsen zur Erzeugung eines auswertbaren Bildes des Fingerreliefs.

Es sind deshalb Anstrengungen dahin gemacht worden, das Licht durch ein anderes Übertragungsmedium zu ersetzen. Beispielsweise geht aus der US-PS 3 622 989 ein Fingerabdruck-Erkennungssystem hervor, bei welchem ein mit einem gemeinsamen Stab verbundener Finger gegen ein Feld von Abtastelektroden gedrückt wird und ein Kontakt zwischen dem Kontaktstab und ausgewählten Abtastelektroden über Rippen der Fingerfläche ausgewählt werden. Die Ausnutzung der Leitfähigkeit der Fingeroberfläche zur Erzeugung einer Stromverteilung ist theoretisch ein vielversprechender Fortschritt. In der Praxis ist es jedoch schwierig, reproduzierbare Ergebnisse zu erzielen. Praktisch kann jede Verunreinigung, wie beispielsweise Feuchtigkeit oder Staub auf dem zu untersuchenden Finger oder auf der Kontaktfläche des Elektrodenfeldes die Stromverteilung verfälschen.

In der älteren US-Patentanmeldung Serial Nr. 170 606, angemeldet am 21. Juli 1980 ist ein Sensor zum Umwandeln des Druckmusters eines Fingerabdrucks in ein Ladungsmuster mittels einer piezoelektrischen Substanz vorgeschlagen. Das Ladungsmuster wird nacheinander durch eine ladungsgekoppelte Vorrichtung (CCD) oder Ladungsübertragungsanordnung in Matrixform, beispielsweise von der Art, wie sie aus Preliminary Data Sheet for a Solid State Image Sensor Array RA 100 × 100, EG & G Reticon, Sunnyvale, California, hervorgeht, gemessen.

Aufgabe der vorliegenden Erfindung ist es, einen Fingerabdrucksensor zum Transformieren der in der epidermalen oder papillaren Struktur des Fingers enthaltenen Information in ein elektrisches Ausgangssignal ohne eine Zwischenumwandlung in eine optische Information anzugeben.

Diese Aufgabe wird durch die im kennzeichnenden Teil des Patentanspruchs 1 angegebenen Merkmale gelöst.

Bevorzugte und vorteilhafte Ausgestaltungen des erfindungsgemäßen Sensors gehen aus den Unteransprüchen 2 bis 19 hervor.

Vorteile der Erfindung sind unter anderem folgende: Es ist ein Fingerabdrucksensor geschaffen, der kleine Dimensionen aufweist, der bezüglich mechanischer Einflüsse intensiv ist und leicht aus kommerziell erhältlichen Komponenten zusammengesetzt werden kann. Der erfindungsgemäße Fingerabdrucksensor kann in Festkörpertechnologie mit integrierten Schaltkreisen ausgeführt werden.

Der erfindungsgemäse Fingerabdrucksensor weist eine hohe Empfindlichkeit und hohe Zuverlässigkeit auf und benötigt eine minimale Leistung.

Die genannte Aufgabe und die vorstehenden Vorteile werden unter anderen Vorteilen, die aus der später folgenden Beschreibung hervorgehen, erfindungsgemäß durch einen Kontaktkörper mit einer Kontaktfläche zur Aufnahme eines zu untersuchenden Fingers, durch ein Feld aus Abtastzellen, das bei dem Kontaktkörper angeordnet ist und je Zelle einen Transistor aufweist, durch einen Schaltkreis zum Messen der Größe des durch verschiedene Transistoren in den Abtastzellen fließenden Stromes, und durch einen Schaltkreis zum Voreinstellen des Arbeitspunktes der Transistoren längs ihrer Strom(I)-/Spannung(V)-Charakteristiken erreicht, in welchen sie als Verstärker wirken. Diese Arbeitspunkte werden entweder direkt oder indirekt durch das topologische Muster eines Fingerabdrucks beeinflußt.

Erfindungsgemäs können deshalb die Arbeitspunkte der Transistoren vor dem Plazieren eines Fingers auf der Kontaktfläche vorhanden sein, und die Änderung in den Arbeitspunkten kann gemessen werden, wenn der Kontaktdruck des Fingers danach ausgeübt wird. Ähnlich können die Arbeitspunkte der Transistoren während der Ausübung eines Kontaktdruckes durch einen Finger auf die Kontaktfläche vorhanden sein. und die Änderung in den Arbeitspunkten kann gemessen werden, wenn der Kontaktdruck entfernt wird.

Die vorliegende Erfindung basiert im wesentlichen auf dem Prinzip, daß der Arbeitspunkt eines Transistorverstärkers durch die Änderung gewisser physikalischer Parameter variiert werden kann, denen er ausgesetzt ist, wie beispielsweise dem Druck, der Umgebungstemperatur und der Umgebungsspannung. Das topologische Muster eines Fingerabdrucks resultiert beim Drücken gegen die Kontaktfläche in entsprechenden Variationen des Druckes und der Temperatur auf der Kontaktfläche. Diese Variationen der Temperatur und des Druckes können durch das Feld aus Sensorzellen durch direkte Änderung des Arbeitspunktes des Transistors in jeder Zelle abgetastet werden oder sie können indirekt durch eine Umwandlung in ein elektrisches Spannungsmuster abgetastet werden. Insbesondere können die Druckvariationen in ein Spannungsmuster durch einen piezoelektrischen Kristall umgewandelt werden,. oder die Temperaturvariationen können in ein Spannungsmuster durch einen pyroelektrischen Kristall umgewandelt werden.

In einem Fall, in dem das Feld aus Sensorzellen direkt entweder auf den Druck oder die Temperatur anspricht, können die Arbeitspunkte der Transistoren durch entweder einen druck- oder temperaturabhängigen Transistor an jeder Zellenstelle in Verbindung mit dem betreffenden Transistor in dieser Stelle variiert werden.

Das Auslesen der Arbeitspunkte der Transistoren in dem Abtastzellenfeld wird vorzugsweise durch eine Anzahl von Zeilenleitern bewirkt, von denen jeder eine erste und zweite Zeilenleitung aufweist, sowie durch eine Anzahl von Spaltenleitern. Ein Zeilenschieberegister mit einem Eingang zum Empfang eines Zeilenschiebesignals und einer Anzahl von Ausgängen ist an die Zeilenleiter gekoppelt. Ein Spaltenschieberegister mit einem Eingang zum Empfang eines Spaltenschiebesignals und einer Anzahl von Ausgängen ist an die Spaltenleiter gekoppelt. Diese Anordnung ermöglicht das Abfragen einer jeden Sensorzelle um seinerseits eine das topologische Muster des angedrückten Fingers anzeigende Auslesung zu ermöglichen.

Für ein volles Verständnis der vorliegenden Erfindung wird auf die folgende Beschreibung der bevorzugten Ausführungsformen der Erfindung anhand der beigefügten Zeichnungen verwiesen. Es zeigen:

Figur 1 eine Seitenansicht eines erfindungsgemäßen Fingerabdrucksensors;

Figur 2 eine Querschnittsansicht eines Teils des Fingerabdrucksensors nach Figur 1;

Figur 3 eine Querschnittsansicht, die ein Detail einer bevorzugten Ausführungsform der vorliegenden Erfindung zeigt;

Figur 4 ein Strom(I)-/Spannungs(V)-Diagramm eines in dem Sensorfeld des erfindungsgemäßen Fingerabdrucksensors verwendeten Transistors;

Figur 5 ein Schaltbild des Abtast- und Ausleseschaltkreises einer bevorzugten Ausführungsform der vorliegenden Erfindung:

Figur 6 ein Schaltbild des Ausleseschaltkreises für eine einzelne Sensorzelle in der Anordnung nach Figur 5;

Figur 7 ein Schaltbild einer Stufe eines mit statischen Flipflops ausgeführten Schieberegisters:

Figur 8 ein topologisches Layout einer Zelle des erfindungsgemäßen Sensorfeldes, bei welchem ein piezoelektrischer oder pyroelektrischer Kristall verwendet ist;

Figur 9 ein Schaltbild einer Sensorzelle, die einen temperatur- oder druckabhängigen Widerstand verwendet; und

Figur 10 ein topologisches Layout einer Zelle des Abtastfeldes nach Figur 9.

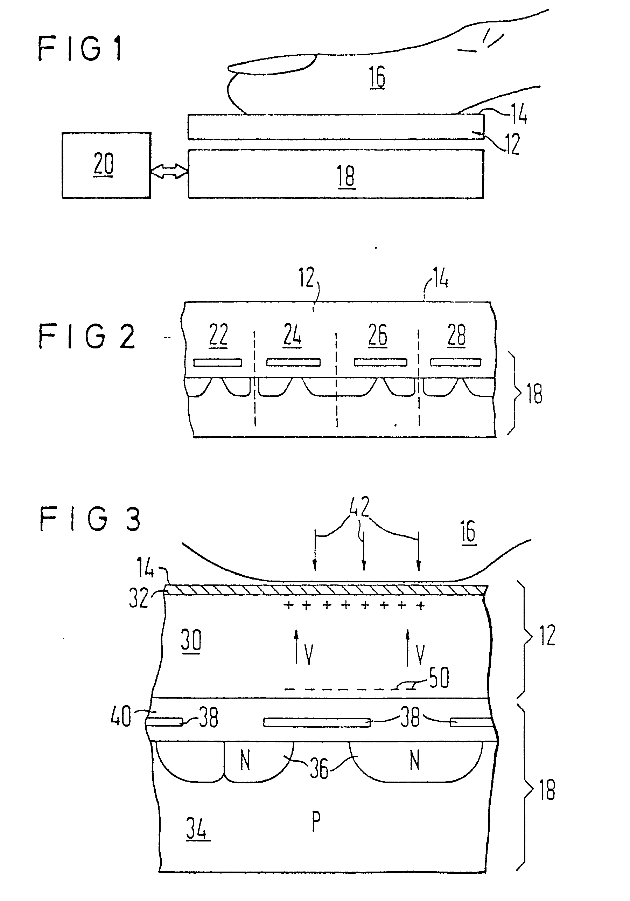

Die bevorzugten Ausführungsformen der vorliegenden Erfindung werden nun unter Bezugnahme auf die Figuren 1 bis 10 der Zeichnungen beschrieben. Identische Elemente in den verschiedenen Figuren sind mit den gleichen Bezugszeichen bezeichnet.

Die grundlegenden Erfordernisse für einen Fingerabdrucksensor liegen darin, die rippenförmige topologische Struktur bzw. den «Fingerabdruck» der Haut über einen Bereich von annähernd 1,5 cm$^2$ mit einer Auflösung in der Größenordnung von 0,1 mm abzutasten. Erfindungsgemäß wird die topologische Struktur abgetastet, indem entweder die Temperatur oder der Druck an einer Fingerabdruck-Kontaktfläche durch Überwachung oder Überprüfung der jeweiligen Operationspunkte eines Feldes oder einer Matrix von Transistoren umgewandelt wird, die neben der Kontaktfläche angeordnet sind. Die Operations-, Betriebs- oder Arbeitspunkte dieser Transistoren werden direkt oder indirekt durch das Druck- oder Temperaturmuster des Fingerabdrucks beeinflußt. Für eine direkte Messung des Druckes oder der Temperatur können die Transistoren selbst druck- oder temperaturabhängige Arbeitspunkte haben oder sie können jeweils in einen Schaltkreis eingeschaltet sein, welcher einen Widerstand aufweist, der einen druck- oder temperaturabhängigen Widerstandswert aufweist. Für die indirekte Messung des Druckes oder der Temperatur können die Transistoren durch Variationen in einer elektrischen Ladung beeinflußt werden, die aus einer piezoelektrischenm oder pyroelektrischen Kristallschicht herrührt. Wenn druck- oder temperaturempfindliche Widerstände verwendet werden, hängen die Arbeitspunkte der Transistoren von den augenblicklichen Werten des Widerstandes ab, die ihrerseits von dem örtlich beschränkten Druck oder der örtlich beschränkten Temperatur abhängen. Wenn eine piezoelektrische oder pyroelektrische Kristallschicht verwendet wird, wandelt diese Schicht einen lokalisierten Druck bzw. eine lokalisierte Temperatur in eine lokalisierte Ladung um, die ihrerseits die Arbeitspunkte der in der Nähe liegenden Transistoren beeinflußt.

Die Figur 1 zeigt einen Fingerabdrucksensor, welcher einen Kontaktkörper 12 enthält, der eine Kontaktfläche 14 zur Aufnahme eines durch einen Finger 16 ausgeübten Kontaktdruckes aufweist. Der Fingerabdruck an der Fingerkuppe bildet ein topologisches Muster, das in ein Informationsmuster umgewandelt wird. welches durch lokalisierte Variationen des Druckes und/oder der Temperatur an der Kontaktfläche gebildet wird. Unmittelbar unterhalb des Kontaktkörpers ist ein integrierter Schaltkreis 18 angeordnet, der ein Feld aus Abtastzellen bildet, von denen jede in der Größenordnung von 0,1 mm$^2$ liegt. Jede Abtastzelle enthält einen Transistor, der einen innerhalb eines Verstärkungsbereiches liegenden Verstärkungsfaktor aufweist, der von dem nächstliegenden Teil des Informationsmusters abhängt.

Der integrierte Schaltkreis 18 ist mit einem ihn betreibenden elektronischen Schaltkreis 20 verbunden, der eine Einrichtung zum Voreinstellen der Arbeitspunkte der erwähnten Transistoren des Abtastzellenfeldes innerhalb ihrer Verstärkungsbereiche enthält, sowie eine Einrichtung zur Messung der Stromstärke oder der Größe des Stromes, die durch jeden Transistor fließt, und zur Ableitung eines elektrischen Ausgangssignals daraus.

Figur 2 zeigt die Struktur der Vorrichtung nach Figur 1 mehr im Detail. Insbesondere ist der integrierte Schaltkreis unter den Kontaktkörper 12 mit einer Anzahl von Feldeffekttransistoren 22, 24, 25 und 28 versehen, die in einer regulären Matrix angeordnet sind und dadurch eine Anzahl oder Vielzahl von Abtastzellen bilden. Die Arbeitspunkte dieser Transistoren werden direkt oder indirekt durch den lokalisierten Druck und/oder die lokalisierte Temperatur beeinflußt, der bzw. die von einem Finger auf die Kontaktfläche 14 ausgeübt wird.

Die in einem Kontaktkörper und einem benachbarten integrierten Schaltkreis enthaltene Konfiguration und enthaltenen annähernden Größen sind in der Figur 3 gezeigt, die eine bevorzugte Ausführungsform der vorliegenden Erfindung zeigt. In dieser Ausführungsform enthält der Kontaktkörper entweder eine piezoelektrische oder pyroelektrische Kristallschicht 30, die zwischen einer metallisierten Schicht 32 und einem integrierten Schaltkreis 18 angeordnet ist, so daß eine Schichtbauweise gegeben ist. Der integrierte Schaltkreis umfaßt ein P-leitendes Substrat 34, in das eine Vielzahl von N-leitenden Regionen oder Bereichen 36 eindiffundiert ist, welche die Source- und Drainelektroden eines Feldes aus N-Kanal-Feldeffekttransistoren bilden. Die Art der Leitfähigkeit kann natürlich umgekehrt sein, so daß P-Kanaltransistoren ausgebildet sind. Über den diffundierten N-Bereichen 36 sind Gateelektroden 38 aus Polysilizium angeordnet und in einer Schicht 40 aus isolierendem Material eingebettet. Beispielsweise kann, wenn das Substrat 34 aus Silizium gebildet ist, die isolierende Schicht aus Siliziumdioxid (SiO$_2$) bestehen. Nötigenfalls kann die obere Fläche der Schicht 40 flach oder plan geschliffen sein, so daß ein inniger Kontakt mit der planaren Fläche der Kristallschicht 30 ausgebildet ist.

Es ist möglich, die Kristallschicht 30 direkt auf der Fläche der isolierenden Schicht 40 aufwachsen zu lassen.

Obwohl dieses Verfahren die Herstellung vereinfacht, kann es die Leistung oder Leistungsfähigkeit der Vorrichtung erniedrigen. weil beispielsweise mit einem piezoelektrischen Kristall der maximale piezoelektrische Effekt eher aus einer monokristallinen Struktur aus Quarz (SiO$_2$) ehalten wird, als mit der polykristallinen Struktur. die normalerweise bei der Herstellung integrierter Schaltkreise erhalten wird.

Die metallisierte Schicht 32 auf der Kristallschicht 30 ist mit Masse verbunden, so daß eine wohldefinierte Grenzbedingung zur Erfassung der induzierten Spannungen hergestellt ist. Diese Schicht bildet die Kontaktfläche 14 für den Finger 16.

Wenn der Finger 16 gegen die Kontaktfläche 14 gedrückt wird, werden an der oberen und unteren Fläche der Kristallschicht 30 lokalisierte Ladungen induziert. Die entsprechenden Spannungen quer über den Kristall, die diesen Ladungen entsprechen, können leicht berechnet werden. Der von dem Finger 16 in Richtung der Pfeile 42 ausgeübte Kontaktdruck p sei 0,5 kg/cm$^{-2}$. Dieser Druck wird erhalten, wenn ein Finger eine totale Kraft von 0,5 kg auf einer Fläche von 2 cm$^2$ ausübt, von der wegen der Rippen nur eine Hälfte mit dem Sensor in Berührung steht. Die induzierte Spannung (V) in einem piezoelektrischen Kristall ist durch

$$V = d \cdot T \cdot h / \varepsilon\varepsilon_0$$

gegeben, in der

d = piezoelektrischer Koeffizient des Kristallmaterials,

T = Spannung oder Belastung im Kristall = p × 9,81,

h = Dicke des Kristalls und

$\varepsilon\varepsilon_0$ = Dielektrizitätskonstante des Kristallmaterials,

bestehend aus dem Produkt aus der relativen Dielektrizitätskonstante des Materials und der absoluten Dielektrizitätskonstante.

Das piezoelektrische Material kann Quarz SiO$_2$ sein, der mit der Fabrikation von integrierten Siliziumschaltkreisen kompatibel ist. In diesem Fall ist d = 2,12 × 10$^{-12}$ Coulomb/N und $\varepsilon\varepsilon_0$ = 3.9, woraus eine induzierte Spannung von 300 mV resultiert. Diese Spannung reicht aus, die Arbeitspunkte der unmittelbar darunter angeordneten Feldeffekttransistoren zu beeinflussen.

Eine andere Möglichkeit besteht darin, ein viel empfindlicheres piezoelektrisches Keramikmaterial zu verwenden, welches aus Bleititanat (PbTiO$_3$), Bleizirkonat (PbZrO$_3$) und Bleinickelniobat (PbNi$_{1/3}$Nb$_{2/3}$O$_3$) besteht. Dieses Material kann von der Fa. Siemens AG, München, Bundesrepublik Deutschland, unter der Handelsmarke VI-BRIT® bezogen werden. Die Verwendung dieses Materials hätte eine induzierte Spannung von mehreren Volts zur Folge.

Für eine temperaturempfindliche Vorrichtung kann das pyroelektrische Material ein im Handel erhältliches Polyvinylidendifluorid (BVDF) sein, das einen Ladungspyrokoeffizienten von 2nCb/cm²°C und einen Spannungspyrokoeffizienten von 1100 V/cm °C aufweist.

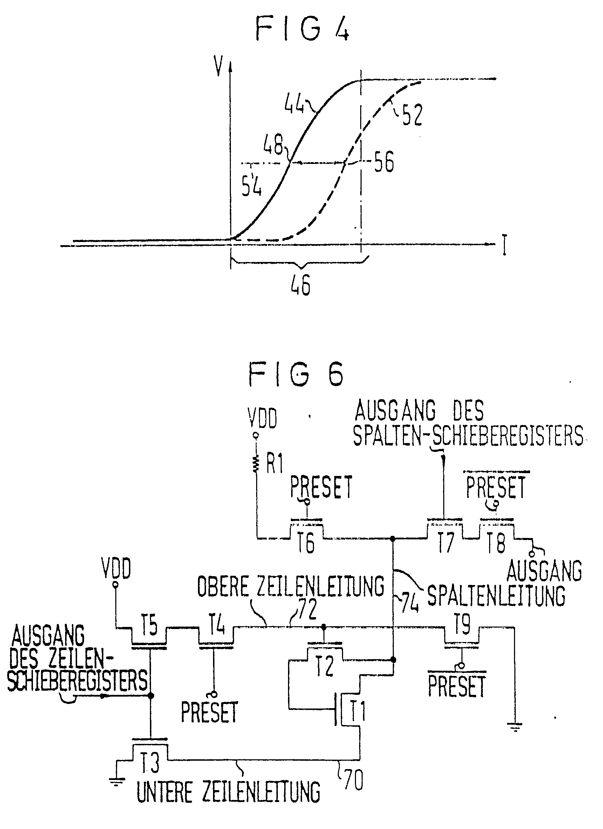

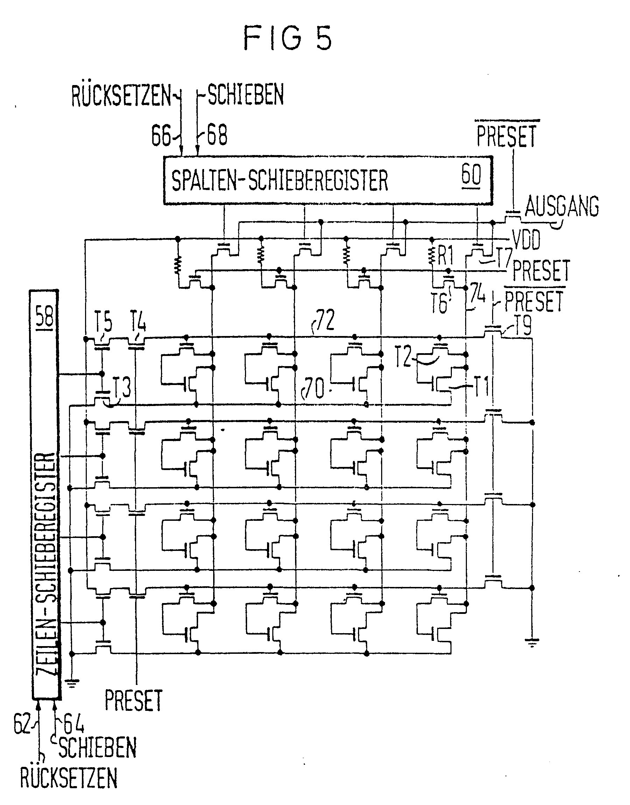

Die Figur 4 zeigt eine typische Strom(I)-/Spannungs(V)-Charakteristik 44 für einen Feldeffekttransistor. Der Verstärkungsbereich des die Charakteristik 44 aufweisenden Transistors liegt in dem Bereich 46. Der Arbeitspunkt 48 wird sowohl durch den Widerstand in dem Source-Drain-Pfad als auch durch die an dem Gate anliegende Spannung bestimmt. Wenn jedoch eine zusätzliche Ladung in der Nähe des Transistorgates vorhanden ist, so wie es durch die negativen Ladungen 50 in Figur 3 dargestellt ist, wird die Strom-Spannungs-Charakteristik des Transistors so verschoben, wie es durch die gestrichelte Linie 52 in Figur 4 dargestellt ist. Eine Folge davon ist, daß der Arbeitspunkt ebenfalls längs einer Linie 54 konstanter Spannung zu einem neuen Punkt 56 verschoben wird. Die resultierende Verschiebung im Stromfluß durch den Transistor kann durch einen Abtastschaltkreis überwacht werden, der von einem Typ sein kann, wie er in den Figuren 5 bis 7 dargestellt ist und im folgenden beschrieben wird.

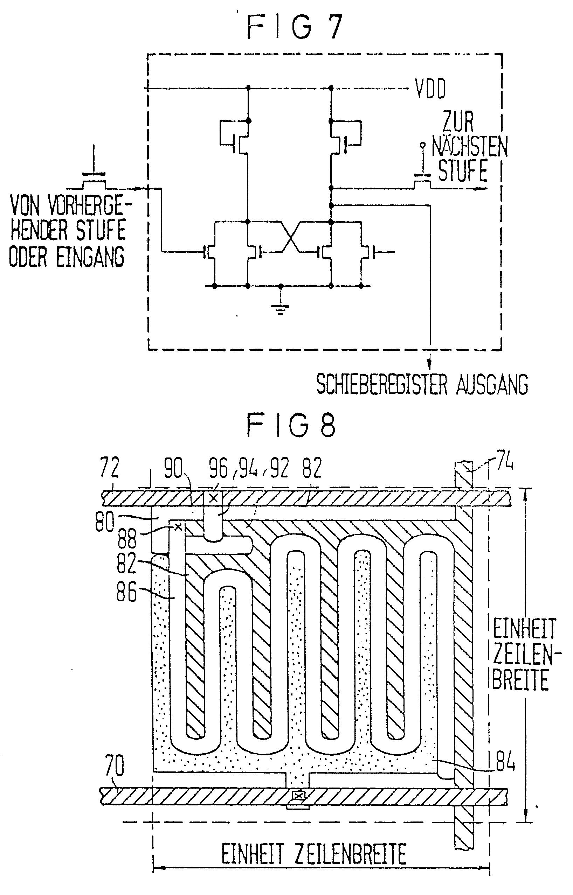

Der Abtastschaltkreis ist als eine Feldstruktur mit zwischen 100 und 200 Elementen auf jeder Seite des Feldes konstruiert, in Abhängigkeit von der erforderlichen Auflösung. Ein solcher Abtastschaltkreis ist in der Figur 5 für den vereinfachten Fall eines 4 × 4-Feldes oder einer 4 × 4-Matrix dargestellt. Der detaillierte Schaltkreis für ein Abtastelement ist in der Figur 6 dargestellt.

Der Schaltkreis nach den Figuren 5 und 6 umfaßt ein Zeilenschieberegister 58 und ein Spaltenschieberegister 60. Das Zeilenschieberegister 58 wird durch ein an einen ersten Eingang 62 angelegtes Signal zurückgesetzt und durch ein an einen zweiten Eingang 64 angelegtes Schiebesignal erhöht. Ähnlich wird das Spaltenschieberegister 60 durch ein an einen ersten Eingang 66 angelegtes Signal rückgesetzt und durch ein an einen zweiten Eingang 68 angelegtes Schiebesignal erhöht. Das Zeilenschieberegister und das Spaltenschieberegister haben jeweils vier Ausgänge (in der dargestellten 4 × 4-Matrix), die aufeinanderfolgend aktiviert werden, wenn das entsprechende Register verschoben wird. Die Ausgänge des Zeilenschieberegisters sind mit den Gates der Transistoren T3 und T5 (siehe Figur 6) verbunden, die eine untere Zeilenleitung 70 und eine obere Zeilenleitung 72 mit Masse bzw. einer Versorgungsspannung VDD verbinden. Jeder Ausgang des Spaltenschieberegisters ist mit dem Gate eines Transistors T7 verbunden, welcher eine Spaltenleitung 74 mit dem Schaltkreisausgang verbindet. Diese Spaltenleitung ist auch über einen Transistor T6 und einen Widerstand R1 mit der Spannung VDD verbunden. Ein Abtasttransistor T1, der so konstruiert ist, wie es in der Figur 3 dargestellt ist, und ein Steuertransistor T2 sind an der Schnittstelle der beiden Zeilenleitungen 70 und 72 und der Spaltenleitung 74 angeordnet. Die Source und der Drain des Abtasttransistors verbinden die Spaltenleitung mit der unteren Zeilenleitung, während die Source und der Drain des Steuertransistors T2 die Spaltenleitung 74 mit dem Gate des zugeordneten Abtasttransistors T1 verbindet. Das Gate des Steuertransistors T2 ist mit der oberen Zeilenleitung verbunden, die entweder auf dem Versorgungsspannungspotential VDD liegt, und zwar wenn der Transistor T4 leitet, oder auf Masse liegt, wenn der Transistor T9 leitet.

Ähnlich ist die Spaltenleitung 74 entweder über den Widerstand R1 mit dem Versorgungsspannungspotential VDD verbunden, und zwar wenn der Transistor T6 leitet, oder mit dem Schaltkreisausgang verbunden, wenn der Transistor T8 leitet. An den Transistoren T4 und T6 liegt gleichzeitig ein Signal PRESET an, während das invertierte Signal PRESET an den Transistoren T8 und T9 anliegt.

Es gibt zwei Verfahren zum Betrieb des Abtastschaltkreises nach den Figuren 5 und 6. Bei einem Verfahren wird der Schaltkreis voreingestellt (preset) während der Finger gegen die Kontaktfläche des Sensors gepreßt wird und die Information wird dann ausgelesen, nachdem der Druck entfernt worden ist. Bei dem anderen Verfahren wird der Schaltkreis in Abwesenheit eines Kontaktdruckes durch den Finger voreingestellt und dann wird die Information ausgelesen, während der Finger gegen die Kontaktfläche gepreßt wird. Diese zwei Verfahren erfordern eine entgegengesetzte Polarität des piezoelektrischen oder pyroelektrischen Materials.

Das erste Verfahren, welches wegen der kürzeren Zeit, während welcher der Finger auf dem Sensor stabil oder ruhig sein muß, als vorzuziehen betrachtet wird, umfaßt die folgenden wesentlichen Schritte. wobei N-Kanal-Feldeffekttransistoren in dem Sensorfeld angenommen sind:

(1) Ein Finger 16 wird gegen die Kontaktfläche 14 eines abtastenden Elements gedrückt, wodurch bzw. welches die nachfolgenden Schritte (2) bis (5) getriggert werden bzw. triggert;

(2) das Spaltenschieberegister und das Zeilenschieberegister werden rückgesetzt;

(3) das Signal PRESET geht hoch;

(4) das Zeilenschieberegister wird aktiviert und schreitet durch alle Zeilen. Wenn jede Zeile aktiviert ist, geht deren obere Zeilenleitung «hoch» und die untere Zielenleitung wird geerdet. Für jede Spalte leitet der Transistor T2 und verbindet Gate und Drain von T1. Das Gate von T1 stellt sich auf eine Spannung ein, die durch die Diodencharakteristik von T1 und den Widerstand R1 definiert ist. Der Wert von R1 wird hinreichend hoch gewählt, so daß T1 gerade leitend ist;

(5) nachdem auf diese Weise all die Abtasttransistoren voreingestellt sind, geht das PRESET-Signal wieder auf einen niedrigen Wert;

(6) der Finger 16 wird von der Kontaktfläche 14 entfernt, wodurch der folgende Schritt (7) getriggert oder ausgelöst wird;

(7) die Zeilen- und Spaltenschiebesignale werden aktiviert, so daß sie schrittweise durch das ganze Feld gehen, Zelle um Zelle der Matrix. Für jede Zelle wird der Strom, der durch den Abtasttransistor T1 fließt, gemessen, der die Sensorausgangsinformation bildet.

Wenn P-Kanal-Feldeffekttransistoren benutzt werden, muß die Polarität des PRESET-Signals umgekehrt werden, so daß die Transistoren T4 und T6 leiten und die Transistoren T8 und T9 blockieren, wenn das PRESET-Signal da ist.

Sowhl die in Figur 5 dargestellten Logik- und Steuerschaltkreise als auch jene Schaltkreise, die zur Erzeugung des Antriebssignals PRESET, zum Verschieben und Rücksetzen, zur Durchführung der vorstehenden Schritte (1) bis (7) notwendig sind, können in dem Halbleiterschaltkreis zusammen mit dem Sensorzellenfeld und zusammen mit den Verstärkerschaltkreisen für das Ausgangssignal integriert werden. Diese Integration wird am besten ausgeführt, wenn das Chip minimaler Konfiguration schon funktioniert.

Das Zeilenschieberegister 58 und Spaltenschieberegister 60 können entweder als dynamische Schieberegister oder als Schieberegister mit statischen Flipflops ausgeführt sein. Für diese Anwendung wird ein Schieberegister, das mit statischen Flipflops ausgeführt ist, wie es in Figur 7 dargestellt ist, bevorzugt. Durch die große Gittergröße der Sensorzellenmatrix werden stringente Erfordernisse bezüglich der Dichte des Layouts vermieden. Anstelle des Zeilen- und Spaltenschieberegisters können auch Dekodierer verwendet werden, die einen wahlfreien Zugriff zu jedem Punkt in der Matrix ermöglichen. Dieser wahlfreie Zugriff könnte von Vorteil sein, wenn eine anspruchsvolle Software zur Erzeugung einer erhöhten oder verbesserten Mustererkennung verwendet wird. Natürlich würden die Dekodierer anstelle des für das Schieberegister vorgesehenen Rücksetz- und Takteingangs eine Anzahl von Adresseingängen erfordern.

Ein geeignetes Layout für die Sensorzelle ist in der Figur 8 dargestellt. Der größte Teil der Fläche dieser Zelle wird von dem großen Abtasttransistor T1 eingenommen. Die übrige Fläche der Zelle wird von Zeilen- und Spaltenleitungen und dem kleinen Steuertransistor T2 eingenommen. Dieses Layout, das eine Zellengröße von 200 × 200 μm hat, was 100 × 100 Elemente auf 2 cm² ergibt, benötigt nur eine sehr konservative oder mäßige minimale Dimension von 10 μm. Eine Verkleinerung auf 150 × 150 μm (was 130 × 130 Elemente auf 2 cm² ergäbe) benötigt immer noch nur 7,5 μm minimale Abmessungen. Bei dem Layout sind, wie dargestellt, für die vertikalen Spaltenleitungen Diffusion oder Implantation verwendet worden und für die horizontalen Zeilenleitungen Polysilizium oder Metall. Dies kann erforderlichenfalls ausgetauscht werden, um die Kapazität der Zeilenleitungen durch Verwendung der Diffusion oder Implantation für sie zu minimieren.

Insbesondere weist das Layout nach Figur 8 ein Substrat 80 auf, welches die untere und obere Zeilenleitung 70 bzw. 72 als Polysilizium oder Metall und eine diffundierte oder implantierte Spaltenleitung 74 trägt. Der diffundierte Bereich erstreckt sich von der vertikalen Spaltenleitung bis zu einer gitterartigen Struktur 82, welche die Drains für die Transistoren T1 und T2 bildet. Ein zweiter diffundierter oder implantierter gitterartiger Bereich 84 ist mit der unteren Zeilenleitung 70 verbunden und bildet die Source des Transistors T1. Eine schlangenförmige Schicht aus Polysilizium 86, welche das Gate des Transistors T1 bildet, ist im Punkt 88 mit einem diffundierten oder implantierten Bereich 90 verbunden, welcher die Source des Transistors T2 bildet. Ein Abschnitt 92 des diffundierten oder implantierten Bereichs 82 bildet den Drain oder die Senke des Transistors T2. Ein bei 96 mit der oberen Zeilenleitung 72 verbundener Streifen 84 aus Polysilizium oder Metall bildet das Gate des Transistors T2.

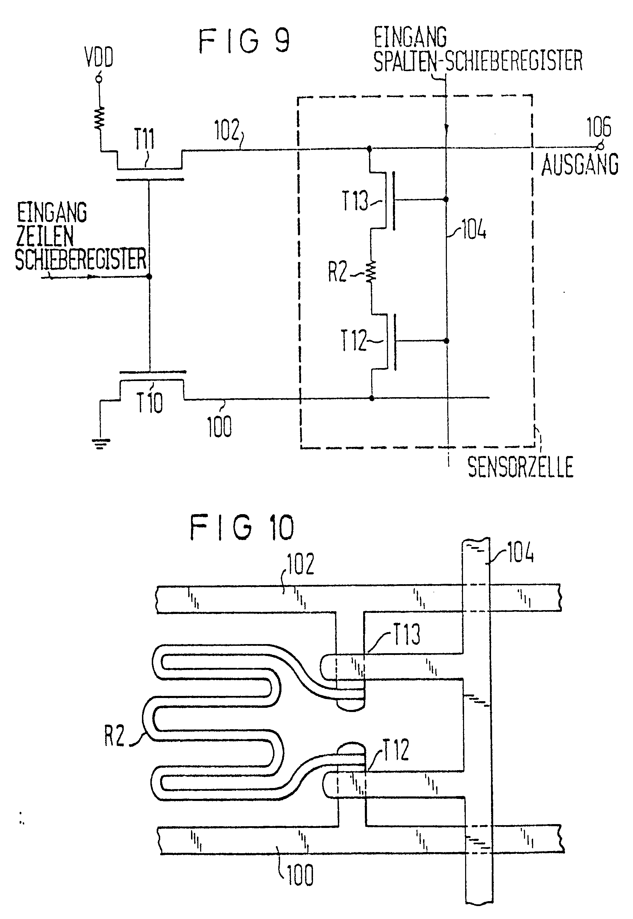

In dem oben beschriebenen Abtastschaltkreis werden Druck- oder Temperaturänderungen an der Kontaktschicht 14 zur indirekten Beeinflussung der Arbeitspunkte der abgetasteten Transistoren benutzt, indem diese Änderungen in das Ladungsmuster umgewandelt wird, wobei eine piezoelektrische bzw. pyroelektrische Schicht 30 verwendet wird. Da Änderungen im Druck und/oder in der Temperatur die Leitfähigkeit eines Transistors in meßbarer Weise ändern, kann der gleiche Typ von Schaltkreis als ein Sensorfeld in dem Fingerabtastsensor ohne Zwischenschaltung einer piezoelektrischen oder pyroelektrischen Kristallschicht verwendet werden. Anstelle von druck-/temperaturabhängigen Transistoren kann auch ein Widerstand verwendet werden, welcher eine starke Abhängigkeit vom Druck und/oder von der Temperatur zeigt. Ein geeigneter Abtastschaltkreis für die Anwendung eines druck- und/oder temperaturabhängigen Widerstandes ist in der Figur 9 dargestellt.

In der Figur 9 sind zwei Zeilenleitungen 100 und 102 über Transistoren T10 und T11 mit Erde bzw. einer Versorgungsspannung VDD verbunden. Eine Spaltenleitung 104 ist mit Gates zweier Transistoren T12 und T13 in einer Zelle verbunden. Wenn sie durch ein Signal an der Spaltenleitung eingeschaltet werden, verbinden die Zellentransistoren T12 und T13 die Zeilen- und Spaltenleitung 100 und 102 über einen druck- und/oder temperaturabhängigen Widerstand R2. Der Strom durch den Widerstand R2 wird durch die Spannung an dem Ausgang 106 reflektiert.

Die Figur 10 zeigt das topologische Layout der in Figur 9 bezeichneten Sensorzelle. Die untere und obere Zeilenleitung 100 bzw. 102 sind diffundierte oder implantierte Bereiche, so wie die Sources oder Quellen und Drains der Transistoren T12 und T13. Der Spaltenleiter 104 und die Gates der Transistoren T12 und T13 bestehen aus Polysilizium oder Metall. Schließlich besteht der druck- und/oder temperaturabhängige Widerstand R2 aus Polysilizium.

Die Verwendung von Festkörpersensoren für die erfindungsgemäße Fingerabdruckverifikation weist gegenüber bekannten Systemen, die auf der Optik basieren, eine Anzahl von Vorteilen auf. Festkörpersensoren sind kompakt, und wenn sie in Masse produziert werden, billig, zuverlässig und robust.

Demgemäß ist ein neuer Sensor für die Fingerabdruckverifikation in Form eines integrierten Schaltkreises gezeigt und beschrieben worden, welcher alle dafür gesuchten Aufgaben und Vorteile erfüllt.

**Patentansprüche**

1. Fingerabdrucksensor zur Transformation des topologischen Musters eines zu untersuchenden Fingers in ein elektrisches Ausgangssignal, der

(a) einen Kontaktkörper (12) mit einer Kontaktfläche (14) zur Aufnahme eines mittels eines Fingers ausgeübten Kontaktdruckes und mit einem Wandlerelement (30) zur Umwandlung des topologischen Musters in ein entsprechendes Informationsmuster aufweist;
gekennzeichnet durch

(b) ein auf der von der Kontaktfläche (14) abgekehrten Seite des Kontaktkörpers (12) angeordnetes Feld aus Abtastzellen (18), wobei jede Zelle einen ersten Transistor (36, 38) mit einer in einem Verstärkungsbereich liegenden Verstärkung, die von dem nächstliegenden Teil des Informationsmusters abhängt;

(c) eine Voreinstelleinrichtung zum Voreinstellen des Arbeitspunktes eines jeden der ersten Transistoren innerhalb seines Verstärkungsbereiches; und

(d) eine Meßeinrichtung zum Messen des durch die ersten Transistoren fließenden Strombetrages und zum Erzeugen des elektrischen Ausgangssignals daraus.

2. Fingerabdrucksensor nach Anspruch 1, dadurch gekennzeichnet, daß die Voreinstelleinrichtung vor Ausübung des Kontaktdruckes betätigbar ist, und daß die Meßeinrichtung die Änderung des Arbeitspunktes mißt, wenn der Kontaktdruck ausgeübt wird.

3. Fingerabdrucksensor nach Anspruch 1, dadurch gekennzeichnet, daß die Voreinstelleinrichtung betätigbar ist, während der Kontaktdruck ausgeübt wird, und daß die Meßeinrichtung die Änderung des Arbeitspunktes mißt, wenn der Kontaktdruck entfernt wird.

4. Fingerabdrucksensor nach einem der vorhergehenden Ansprüche, dadurch gekennzeichnet, daß das Informationsmuster ein Druckmuster ist.

5. Fingerabdrucksensor nach einem der vorhergehenden Ansprüche, dadurch gekennzeichnet, daß das Informationsmuster ein Temperaturmuster ist.

6. Fingerabdrucksensor nach einem der vorhergehenden Ansprüche, dadurch gekennzeichnet, daß das Informationsmuster ein elektrisches Spannungsmuster ist.

7. Fingerabdrucksensor nach Anspruch 6, dadurch gekennzeichnet, daß das Wandlerelement das topologische Muster in ein als Zwischenmuster dienendes Druckmuster und dieses Druckmuster in ein elektrisches Spannungsmuster umwandelt.

8. Fingerabdrucksensor nach Anspruch 7, dadurch gekennzeichnet, daß das Wandlerelement eine Anzahl von ohmschen Abtastwiderständen aufweist, von denen jeder einen druckabhängigen Widerstand aufweist und mit wenigstens einem der Transistoren verbunden ist, um das als Zwischenmuster dienende Druckmuster in das elektrische Spannungsmuster umzuwandeln.

9. Fingerabdrucksensor nach Anspruch 7 oder 8, dadurch gekennzeichnet, daß das Wandlerelement eine piezoelektrische Schicht zum Umwandeln des als Zwischenmuster dienenden Druckmusters in das elektrische Spannungsmuster aufweist, wobei die piezoelektrische Schicht eine nahe bei der Kontaktfläche liegende obere Fläche und eine nahe dem Abtastzellenfeld liegende untere Fläche oder Bodenfläche aufweist.

10. Fingerabdrucksensor nach Anspruch 6, dadurch gekennzeichnet, daß das Wandlerelement das topologische Muster in ein als Zwischenmuster dienendes Temperaturmuster und dieses in das elektrische Spannungsmuster umwandelt.

11. Fingerabdrucksensor nach Anspruch 10, dadurch gekennzeichnet, daß das Wandlerelement eine Anzahl von ohmschen Abtastwiderständen aufweist, von denen jeder einen temperaturabhängigen Widerstand aufweist und mit wenigstens einem der Transistoren verbunden ist, um das als Zwischenmuster dienende Temperaturmuster in das elektrische Spannungsmuster umzuwandeln.

12. Fingerabdrucksensor nach Anspruch 10, dadurch gekennzeichnet, daß das Wandlerelement eine pyroelektrische Schicht zum Umwandeln des als Zwischenmuster dienenden Temperaturmusters in ein elektrisches Spannungsmuster aufweist, wobei die pyroelektrische Schicht eine nahe bei der Kontaktfläche liegende obere Fläche und eine nahe bei dem Abtastzellenfeld liegende untere Fläche oder Bodenfläche aufweist.

13. Fingerabdrucksensor nach einem der vorhergehenden Ansprüche, dadurch gekennzeichnet, daß das Sensorzellenfeld eine Anzahl von Zeilenleitern und eine Anzahl von Spaltenleitern aufweist, wobei jeder Zeilenleiter eine erste und zweite Zeilenleitung aufweist, wobei jede Sensorzelle zusätzlich zu dem ersten Transistor einen zweiten Transistor aufweist, wobei Source und Drain des ersten Transistors eine der Spaltenleiter mit der ersten Zeilenleitung eines jeden Zeilenleiters verbindet und Source und Drain des zweiten Transistors den Spaltenleiter mit dem Gate des ersten Transistors verbindet, wobei das Gate des zweiten Transistors mit der zweiten Zeilenleitung des Zeilenleiters verbunden ist, wobei die erste Zeilenleitung über einen ersten Zeilenleiterschalter mit Erde verbunden ist, wobei die zweite Zeilenleitung über einen zweiten Zeilenleiterschalter mit einer ersten Spannungsquelle verbunden ist, und wobei der Spaltenleiter über einen ersten Spaltenleiterschalter mit einer zweiten Spannungsquelle und über einen zweiten Spaltenleiterschalter mit einem Terminal oder Endanschluß verbunden ist, wobei das Terminal das elektrische Ausgangssignal liefert.

14. Fingerabdrucksensor nach Anspruch 13, dadurch gekennzeichnet, daß der erste und zweite Zeilenleiterschalter und der erste und zweite Spaltenleiterschalter Schalttransistoren sind.

15. Fingerabdrucksensor nach Anspruch 13 oder 14, dadurch gekennzeichnet, daß ein Zeilenschieberegister mit einem Eingang zum Empfang eines Zeilenschiebesignals und einer Anzahl von Ausgängen vorgesehen ist. von denen jeder die ersten und zweiten Zeilenleiterschalter eines der Zeilenleiter steuert, und daß ein Spaltenschieberegister mit einem Eingang zum Empfang eines Spaltenschiebesignals und einer Anzahl von Ausgängen vorgesehen ist, von denen jeder einen der Spaltenleiterschalter steuert.

16. Fingerabdrucksensor nach Anspruch 15, dadurch gekennzeichnet, daß die Schieberegister als dynamische Schieberegister ausgeführt sind.

17. Fingerabdrucksensor nach Anspruch 15, dadurch gekennzeichnet, daß die Schieberegister als statische Flipflops ausgeführt sind.

18. Fingerabdrucksensor nach einem der Ansprüche 15 bis 17, dadurch gekennzeichnet, daß die Voreinstelleinrichtung Voreinstellzeilenleiterschalter und Voreinstellspaltenleiterschalter aufweist, wobei jeder Voreinstellzeilenleiterschalter in eine der zweiten Zeilenleitungen zwischen den Gates der zweiten Transistoren und des zweiten Zeilenleiterschalters eingefügt, und wobei jeder Voreinstellspaltenleiterschalter zwischen einem der Spaltenleiter und der zweiten Spannungsquelle geschaltet ist.

19. Fingerabdrucksensor nach einem der Ansprüche 13 bis 18. dadurch gekennzeichnet, daß die erste und zweite Spannungsquelle identisch sind.

## Claims

1. Fingerprint sensor for the transformation of the topological pattern of a finger to be examined inbto an electric output signal. which

(a) exhibits a contact body (12) with a contact surface (14) for receiving a contact pressure exerted by means of a finger and with a transducer element (30) for converting the topological pattern into a corresponding information pattern, characterized by

(b) an array of scanning cells (19) arranged on the side of the contact body facing away from the contact surface (14), each cell containing a first transistor (36, 38) with a gain which is within a gain range and which depends on the nearest part of the information pattern;

(c) a presetting device for presetting the operating point of each of the first transistors within its gain range; and

(d) a measuring device for measuring the amount of current flowing through the first transistors and for generating from this the electric output signal.

2. Fingerprint sensor according to Claim 1, characterized in that the presetting device can be actuated before exertion of the contact pressure, and that the measuring device measures the change in the operating point when the contact pressure is exerted.

3. Fingerprint sensor according to Claim 1, characterized in that the presetting device can be actuated as the contact pressure is being exerted and that the measuring device measures the change in the operating point when the contact pressure is removed.

4. Fingerprint sensor according to one of the preceding claims. characterized in that the information pattern is a pressure pattern.

5. Fingerprint sensor according to one of the preceding claims; characterized in that the information pattern is a temperature pattern.

6. Fingerprint sensor according to one of the preceding claims, characterized in that the information pattern is an electric voltage pattern.

7. Fingerprint sensor according to Claim 6, characterized in that the transducer element converts the topological pattern into a pressure pattern used as intermediate pattern and converts this pressure pattern into an electric voltage pattern.

8. Fingerprint sensor according to Claim 7. characterized in that the transducer element exhibits a number of ohmic scanning resistors each of which exhibits a pressure-dependent resistor and is connected to at least one of the transistors to convert the pressure pattern used as intermediate pattern into the electric voltage pattern.

9. Fingerprint sensor according to Claim 7 or 8. characterized in that the transducer element exhibits a piezoelectric layer for converting the pressure pattern used as intermediate pattern into the electric voltage pattern. the piezoelectric layer exhibiting an upper surface which is close to the contact surface and a lower or bottom surface which is close to the scanning cell array.

10. Fingerprint sensor according to Claim 6, characterized in that the transducer element converts the topological pattern into a temperature pattern used as intermediate pattern and converts the latter into the electric voltage pattern.

11. Fingerprint sensor according to Claim 10. characterized in that the transducer element exhibits a number of ohmic scanning resistors, each of which exhibits a temperature-dependent resistance and is connected to at least one of the transistors to convert the temperature pattern used as intermediate pattern into the electric voltage pattern.

12. Fingerprint sensor according to Claim 10. characterized in that the transducer element exhibits a pyroelectric layer for converting the temperature pattern used as intermediate pattern into an electric voltage pattern, the pyroelectric layer exhibiting an upper surface which is close to the contact surface and a lower surface or bottom surface which is close to the scanning cell array.

13. Fingerprint sensor according to one of the preceding claims, characterized in that the sensor cell array exhibits a number of row conductors and a number of column conductors, each row conductor exhibiting a first and second row line, each sensor cell exhibiting a second transistor additionally to the first transistor, source and drain of the first transistor connecting one of the column conductors to the first row line of each row conductor and source and drain of the second transistor connecting the column conductor to the gate of the first transistor. the gate of the second transistor being connected to the second row line of the row conductor. the first row line being connected to earth via a first row conductor switch, the second row line being connected to a first voltage source via a second row conductor switch, and the column conductor being connected via a first column conductor switch to a second voltage source and via a second column conductor switch to a terminal or terminating connection, the terminal supplying the electric output signal.

14. Fingerprint sensor according to Claim 13, characterized in that the first and second row conductor switch and the first and second column conductor switch are switching transistors.

15. Fingerprint sensor according to Claim 13 or 14, characterized in that a row shift register with an input for receiving a row shift signal and a number of outputs is provided, each of which controls the first and second row conductor switches of each of the row conductors, and that a column shift register with an input for receiving a column shift signal and a number of outputs is provided, each of which controls one of the column conductor switches.

16. Fingerprint sensor according to Claim 15, characterized in that the shift registers are constructed as dynamic shift registers.

17. Fingerprint sensor according to Claim 15, characterized in that the shift registers are constructed of static flip flops.

18. Fingerprint sensor according to one of Claims 15 to 17, characterized in that the presetting device exhibits presetting row conductor switches and presetting column conductor switches, each presetting row conductor switch being inserted into one of the second row lines between the gates of the second transistors and the second row conductor switch and each presetting column conductor switch being connected between one of the column conductors and the second voltage source.

19. Fingerprint sensor according to one of Claims 13 to 18. characterized in that the first and second voltage source are identical.

**Revendications**

1. Détecteur d'empreintes digitales servant à transformer le profil topologique d'un doigt à examiner en un signal électrique de sortie, et comportant

(a) un corps de contact (12) muni d'une surface de contact (14) servant à recevoir une pression de contact exercée à l'aide d'un doigt, et un élément transducteur (30) servant à convertir le profil topologique en un profil correspondant d'informations; caractérisé par

(b) un panneau disposé sur la face du corps de contact (12), tournée à l'opposé de la surface de contact (14) et constitué par des cellules d'exploration (18), dont chacune comporte un premier transistor (36, 38) possédant une amplification située dans une plage d'amplification dépendant de la partie la plus proche du profil d'informations;

(c) un dispositif de préréglage servant à prérégler le point de fonctionnement de chacun des premiers transistors à l'intérieur de sa gamme d'amplification;

(d) un dispositif de mesure servant à mesurer la valeur du courant traversant les premiers transistors et à produire. à partir de cette valeur, le signal électrique de sortie.

2. Détecteur d'empreintes digitales suivant la revendication 1, caractérisé par le fait que le dispositif de préréglage peut être actionné avant l'application de la pression de contact et que le dispositif de mesure mesure la variation du point de fonctionnement, lorsque la pression de contact est appliquée.

3. Détecteur d'empreintes digitales suivant la revendication 1. caractérisé par le fait que le dispositif de préréglage peut être actionné alors que la pression de contact est appliquée, et que le dispositif de mesure mesure la variation du point de fonctionnement lorsque la pression de contact est supprimée.

4. Détecteur d'empreintes digitales suivant l'une des revendications précédentes, caractérisé par le fait que le profil d'informations est un profil de pression.

5. Détecteur d'empreintes digitales suivant l'une des revendications précédentes, caractérisé par le fait que le profil d'informations est un profil de température.

6. Détecteur d'empreintes digitales suivant l'une des revendications précédentes, caractérisé par le fait que le profil d'informations est un profil de tension électrique.

7. Détecteur d'empreintes digitales suivant la revendication 6, caractérisé par le fait que l'élément transducteur convertit le profil topologique en un profil de pression utilisé comme profil auxiliaire et que ce profil de pression est converti en un profil de tension électrique.

8. Détecteur d'empreintes digitales suivant la revendication 7, caractérisé par le fait que l'élément transducteur possède un certain nombre de résistances ohmiques d'exploration, dont chacune possède une valeur résistive dépendant de la pression et est reliée à au moins l'un des transistors, de manière à convertir le profil de pression utilisé comme profil intermédiaire en un profil de tension électrique.

9. Détecteur d'empreintes digitales suivant la revendication 7 ou 8, caractérisé par le fait que l'élément transducteur comporte une couche piézoélectrique servant à convertir le profil de pression utilisé comme profil intermédiaire en le profil de tension électrique, la couche piézoélectrique possédant une surface supérieure située à proximité de la surface de contact, et une surface inférieure ou surface de base située à proximité du panneau de cellules d'exploration.

10. Détecteur d'empreintes digitales suivant la revendication 6, caractérisé par le fait que l'élément transducteur convertit le profil topologique en un profil de température utilisé comme profil intermédiaire et convertit ce profil en le profil de tension électrique.

11. Détecteur d'empreintes digitales suivant la revendication 10, caractérisé par le fait que l'élément transducteur possède un certain nombre de résistances ohmiques d'exploration, dont chacune possède une valeur résistive dépendant de la température et est reliée à au moins l'un des transistors, de manière à convertir le profil de température utilisé comme profil intermédiaire en le profil de tension électrique.

12. Détecteur d'empreintes digitales suivant la revendication 10, caractérisé par le fait que l'élément transducteur possède une couche pyroélectrique servant à convertir le profil de température utilisé comme profil intermédiaire en un profil de tension électrique, la couche pyroélectrique comportant une surface supérieure proche de la surface de contact et une surface inférieure ou surface de base proche du panneau des cellules d'exploration.

13. Détecteur d'empreintes digitales suivant l'une des revendications précédentes, caractérisé par le fait que le panneau de cellules du détecteur comporte un certain nombre de conducteurs de lignes et un certain nombre de conducteurs de colonnes, que chaque conducteur de ligne comporte des premières et secondes lignes, que chaque cellule du détecteur comporte un second transistor en plus du premier transistor, que la source et le drain du premier transistor relient l'un des conducteurs de colonnes à la première ligne de chaque conducteur de ligne et que la source et le drain du second transistor relient le conducteur de colonne à la grille du premier transistor, que la grille du second transistor est reliée à la seconde ligne du conducteur de ligne, que la première ligne du conducteur de ligne est reliée à la masse par l'intermédiaire d'un premier interrupteur du conducteur de ligne, que la seconde ligne du conducteur de ligne est reliée à une première source de tension par l'intermédiaire d'un second interrupteur du conducteur de ligne, et que le conducteur de colonne est relié par l'intermédiaire d'un premier interrupteur du conducteur de colonne à une seconde source de tension et par l'intermédiaire d'un second interrupteur du conducteur de colonne à une borne ou à un raccord d'extrémité, la borne délivrant le signal électrique de sortie.

14. Détecteur d'empreintes digitales suivant la revendication 13, caractérisé par le fait que les premier et second interrupteurs des conducteurs de lignes et les premier et second interrupteurs des conducteurs de colonnes sont des transistors de commutation.

15. Détecteur d'empreintes digitales suivant la revendication 13 ou 14, caractérisé par le fait qu'il est prévu un registre à décalage de lignes comportant une entrée servant à recevoir un signal de décalage de lignes et un certain nombre de sorties, dont chacune commande respectivement les premier et second interrupteurs de l'un des conducteurs de lignes, et qu'il est prévu un registre à décalage de colonnes comportant une entrée servant à recevoir un signal de décalage de colonnes et un certain nombre de sorties, dont chacune commande l'un des interrupteurs des conducteurs de colonnes.

16. Détecteur d'empreintes digitales suivant la revendication 15, caractérisé par le fait que les registres à décalage sont réalisés sous la forme de registres à décalage dynamiques.

17. Détecteur d'empreintes digitales suivant la revendication 15, caractérisé par le fait que les registres à décalage sont réalisés sous la forme de bascules bistables statiques.

18. Détecteur d'empreintes digitales suivant l'une des revendications 15 à 17, caractérisé par le fait que le dispositif de préréglage comporte des interrupteurs des conducteurs de lignes de préréglage et des interrupteurs des conducteurs de colonnes de préréglage, que chaque interrupteur de conducteur de ligne de préréglage est inséré dans l'une des secondes lignes des conducteurs de lignes entre les grilles des seconds transistors et le second interrupteur des conducteurs de lignes, et que chaque interrupteur des conducteurs de colonnes de préréglage est branché entre l'un des conducteurs de colonnes et la seconde source de tension.

19. Détecteur d'empreintes digitales suivant l'une des revendications 13 à 18, caractérisé par le fait que les première et seconde sources de tension sont identiques.

F I G 1

16

14

12

20

18

F I G 2

12

14

22    24    26    28

18

F I G 3

42

16

14

32

+ + + + + + + + +

30

↑V    ↑V

50

40    38    38

N    36    N

34    P

18

# FIG 4

# FIG 6

# FIG 5

# FIG 7

VDD

ZUR
NÄCHSTEN
STUFE

VON VORHERGE-
HENDER STUFE
ODER EINGANG

SCHIEBEREGISTER AUSGANG

# FIG 8

72    90    96,94,92  82    74

80

88

82

86

EINHEIT
ZEILEN-
BREITE

70    84

EINHEIT ZEILENBREITE

## FIG 9

VDD

T11

102

EINGANG
SPALTEN-SCHIEBEREGISTER

106
AUSGANG

T13

R2

104

EINGANG
ZEILEN
SCHIEBEREGISTER

T12

T10

100

SENSORZELLE

## FIG 10

102

104

T13

R2

T12

100